# EUROPEAN PATENT APPLICATION

(11) **EP 2 471 444 A1**
(43) Date of publication of application: **04.07.2012**
(21) Application number: 11001422.2
(22) Date of filing: 21.02.2011
(51) Int. Cl.: A61B 5/024, A61B 5/00

(54) **Wireless optical pulsimetry system for a healtcare environment**

(30) Priority: 28.12.2010 US 979651
(71) Applicant: YIP Inc., Chien-Chen Kao hsiung (TW)
(72) Inventor: Liu, Raymond, Chien-Chen Kao hsiung (TW); Kao, Wei-Fong, Taipei (TW)
(74) Representative: Wagner, Bernhard Peter

(57) **Abstract**

The present invention provides a wireless optical pulsimetry system for a healthcare environment comprising a pulse sensor, a USB receiver, and a software. The pulse rate and location information are transmitted from the pulse sensor to the monitoring computer in order to know physiological information of a user in healthcare facilities..

## Description

### FIELD OF THE INVENTION

The present invention relates to a wireless optical pulsimetry system for a healthcare environment, particularly to one which can real-time monitor location and physiological information of users and which can alarm when abnormality of the information occurs.

### BACKGROUND OF THE INVENTION

The heart rate is an important sign, which reflects human physiological states. In the past, the method of monitoring heart rate is to use electrocardiogram analysis or pulse detection. Since electrocardiogram analysis can provide more information of cardiac activities that helps doctors to find the root cause of a disease, it is a dominant diagnostic tool used widely in hospitals. In most cases, pulse rate is equivalent to the heart rate calculated from electrocardiogram. Therefore using a blood oximetry or a blood pressure monitor to measure the pulse rate is a good substitute for using electrocardiogram to evaluate personal heath.

There are many wireless medical devices developed in recent years, such as wireless blood pressure monitor, wireless oximetry, wireless electrocardiographic machine, etc. By combining the wireless technology with miniaturized technology, medical devices become portable. A patient who wears a wireless medical device would no longer be restrained in a specific area nearby the medical equipment. That means it extends the range of his activity. The wireless medical device can transmit patients' physiological information to hospital via wireless and Internet for diagnosis. In general, 2.4GHz radio band (ISM band, Industrial, Scientific and Medical) is used for wireless communication in different protocol (WiFi, Bluetooth, Zigbee, etc.).

Telemedicine, which is defined as transmitting physiological data from local medical device to remote monitor via wireless and Internet, has been developed for several years. One of the advantages of the telemedical device is to first line screen for abnormal patients. The early detection of the patients' condition is always a major concern in healthcare facilities to prevent from deterioration in patients. A controversy in such a case is that there was no enough medical devices to real-time monitor vital signs, such as electrocardiography device (EKG), on a large scale due to its high costs. Compared to EKG, the human pulse rate detection by plethysmographic sensor becomes popular and cheap in recent years. For that matter, developing a pulse detection system can make it possible to real-time monitor patients' health on a large scale with a reasonable cost.

### BRIEF DESCRIPTION OF THE DRAWINGS

Fig. 1 shows a top view of the pulse sensor of the present invention.
Fig. 2A is a top view of the optical finger probe worn by a user of the present invention.
Fig. 2B is a front view of the optical finger probe of the present invention.
Fig. 2C is a cross-section of the top view of soft tip of the optical finger probe of the present invention.
Fig. 3 is a top view of the USB receiver of the present invention.
Fig. 4 is a block diagram of the wireless optical pulsimetry system for a healthcare environment of the present invention.
Fig. 5 is a schematic view of the wireless optical pulsimetry system used in a healthcare environment of the present invention.
Fig. 6 is a block diagram of the pulse sensor of the present invention.
Fig. 7 is a block diagram of the USB receiver of the present invention.
Fig. 8 is a flowchart of physiological signal processing software of the present invention wherein the physiological signal is a pulse signal.

### SUMMARY OF THE INVENTION

In accordance with the present invention, a wireless optical pulsimetry system is used for real-time monitoring human pulse rates on a large scale. In most healthcare facilities, patient monitors are only allocated for a small portion of patients since it is hard for nurses to keep tracking of all patients who are ambulatory in the facility. In the present invention, anyone who wears a pulse sensor can be monitored anywhere in the healthcare facilities within wireless optical pulsimetry system coverage. A ring-like optical finger probe is designed for long-tenn wearing. A flexible coiled cable makes the pulse sensor fit for any size of user's hand. In the present invention, the wireless optical pulsimetry system can automatically update and display the pulse rate and location information of users on a monitoring computer for reducing caretakers' workload. The wireless optical pulsimetry system can easily integrate with other Zigbee-base wireless medical devices, such as a multi vital signs recorder described in the present invention. In contrast to the past, the wireless optical pulsimetry system in the present invention is more applicable for long-term and large-scale vital sign monitoring.

According to the present invention, there is provided a wireless optical pulsimetry system for a healthcare environment comprising:
a pulse sensor for measuring a pulse rate of a user to transmit a pulse signal;
a USB receiver for receiving the pulse signal wirelessly from one or more pulse sensors and transmitting localization information of a user who wears the pulse sensor to a monitoring computer by transmitting means; and
a monitoring software running on the monitoring computer for displaying the pulse rate and the localization information of a user by display means.

According to the present invention, preferably the pulse sensor comprises:
an optical finger probe is used for collecting a pulsatile signal of the user;
a 4-stage pulsatile signal amplifier circuit for amplifying the pulsatile signal obtained from the optical finger probe;
a microprocessor for processing the pulsatile signal, calculating a pulse rate from the pulsatile signal, and transmitting the pulse rate; and
a wireless transceiver module for receiving the pulse rate from the microprocessor and for wireless transmitting the pulse rate to the USB receiver.

According to the present invention, preferably the optical finger probe comprises:
a coiled cable is retractable and flexible for fitting different hand sizes of the user;
a soft tip worn on a finger of the user as a ring for measuring the pulsatile signal of the user; and
a adaptor for fixing the coiled cable on the pulse sensor.

According to the present invention, preferably the soft tip comprises:
an optical sensing apparatus functioning as a plethysmographic sensor consisting of light emitting and detecting elements;
a well providing a space for the optical sensing apparatus embedded;
a strap with bulged stripe for adjusting the ring size of the soft tip;
a plastic buckle for fixing the strap;
a latch for tying up the strap overhung; and
a curved structure for fitting a shape of the finger to enhance the contact between the optical sensing apparatus and the finger.

According to the present invention, preferably the optical sensing apparatus provides real-time pulsatile signal to the 4-stage pulsatile signal amplifier circuit by means of sensing infrared absorption changes due to vascular volume change in the finger.

According to the present invention, preferably collecting of pulse rate wirelessly from one or more pulse sensors is based on Zigbee technology.

According to the present invention, preferably frame data of the Zigbee technology includes a sensor number of the pulse sensor and the pulse rate data.

According to the present invention, preferably the transmitting means comprises:
a pulse sensor for sending the pulse rate to any USB receiver in its wireless coverage;
a USB receiver for receiving the pulse rate from the pulse sensor and using an embedded wireless signal strength indicator to estimate the distance between the pulse sensor and the USB receiver; and
a USB extender or wireless repeater for transmitting the information from the USB receiver to the monitoring computer.

According to the present invention, preferably the display means comprise:
a display software for displaying the pulse rate data, location information, movement of the pulse sensor, status of a wireless signal, and profile of the user;
an adjustable audible and visual alarm for raising the user attention when the pulse rate data exceeds a pre-set limit or ; and
a naming function device for setting the localization information of the USB receiver.

According to the present invention, preferably the healthcare environment includes a hospital, nursing home, home, or other healthcare facilities.

According to the present invention, the wireless optical pulsimetry system for a healthcare environment further comprises a first-line screening function used in healthcare environments for finding a user with abnormal pulse rate data that might be caused by complex physiological abnormality.

According to the present invention, the wireless optical pulsimetry system for a healthcare environment further comprises a multi vital signs recorder for collecting and transmitting multiple vital signs to the monitoring computer.

According to the present invention, preferably the wireless optical pulsimetry system is a real-time wireless optical pulsimetry system for monitoring the user in a real-time manner.

### DETAILED DESCRIPTION OF THE INVENTION

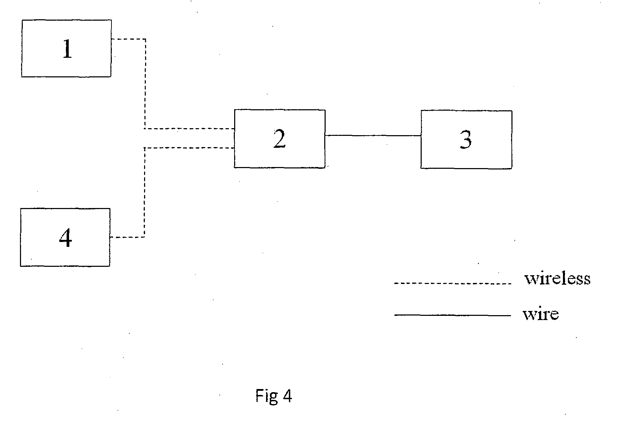

The wireless optical pulsimetry system for a healthcare environment of the present invention, as shown in Fig. 4, comprises a pulse sensor 1, a USB receiver 2, a monitoring computer 3, a multi vital signs recorder 4. Human pulse rate is collected from the pulse sensor 1 and then automatically transmitted to the USB receiver 2 via wireless transmission. Other vital signs, such as temperature and blood pressure, are manually inputted into a multi vital signs recorder and transmitted to the USB receiver 2 via wireless transmission. A plurality of the wireless USB receivers 2 can transmit vital signs and location information from one or more pulse sensors or multi vital sign recorders to the monitoring computer 3 through a wire or wireless communication. The monitoring computer 3 is connected to hospital information system (HIS) or Internet. Caretakers or doctors can obtain and access the physiological data of the monitoring computer 3 via HIS or Internet. The wireless optical pulsimetry system can be used in healthcare facilities, hospitals, nursing homes, home, or other places in which a lot of patients need constant care or monitoring. The wireless optical pulsimetry system can function as a first-line screening in healthcare environments to find out patients with abnormal pulse rate data that might be caused by complex physiological abnormality. The pulse data and location information of every user can be displayed in the monitoring computer 3. In the software, user's profile and pulse rate limits can be set. There is also a naming function for setting the localization information of the USB receiver 2.

The advantages of the wireless optical pulsimetry system for a healthcare environment of the present invention are that (1) it helps caretakers to screen high-risk patients by audio or visual alarm. Then, the screened patients can be examined and diagnosed in detail, and (2) it can increase the safety of the patients and decrease the workload in a routine vital sign measurement; moreover, it changes traditional healthcare procedure. (3) The portable design of the device would not restrain users from walking or other location changes. (4) The design of wireless roaming achieves non-interrupt and real-time sensing for the pulse signal. (5) Within the wireless optical pulsimetry system coverage, careless indoor areas, such as toilet or basement, can receive the pulse signals from the pulse sensor to monitor. (6) A forbidden area can be set in the software. The system will alarm if users enter the preset forbidden area. (7) Different alarm limit can be set in the software for each user. (8) An emergency button on the pulse sensor provides users an additional way to call for help. (9) Using Zigbee, a power saving protocol of wireless communication, pulse sensor can continuously works for more than 5 days with a 550 mAh Li-Polymer battery. (10) All data collected in this system can be saved in the monitoring computer for reference.

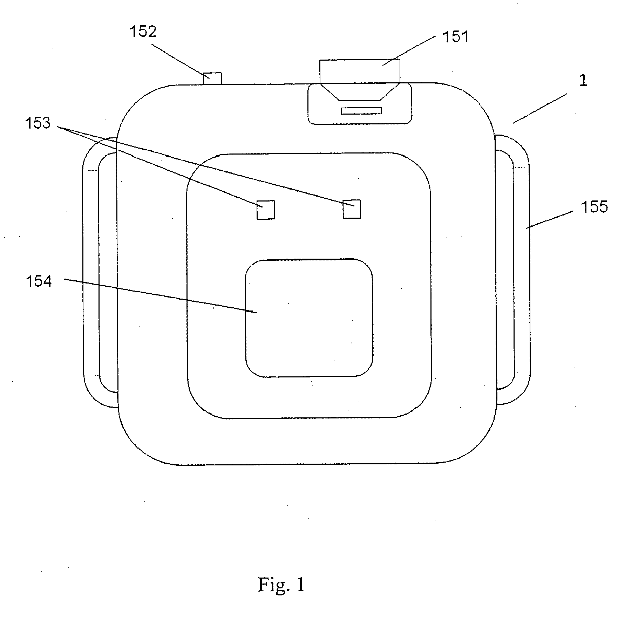

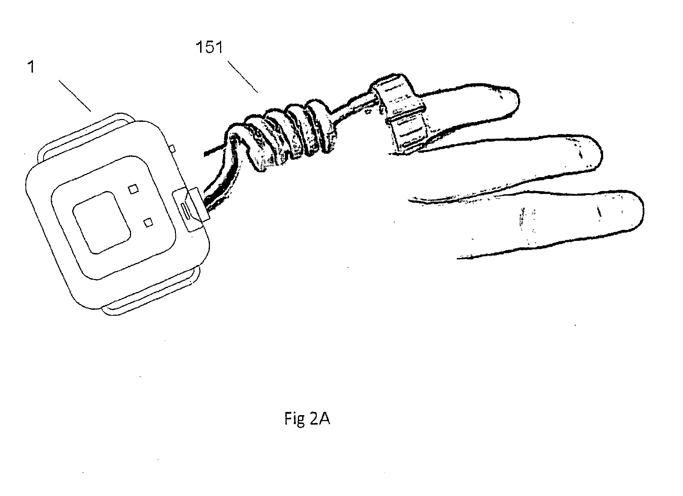

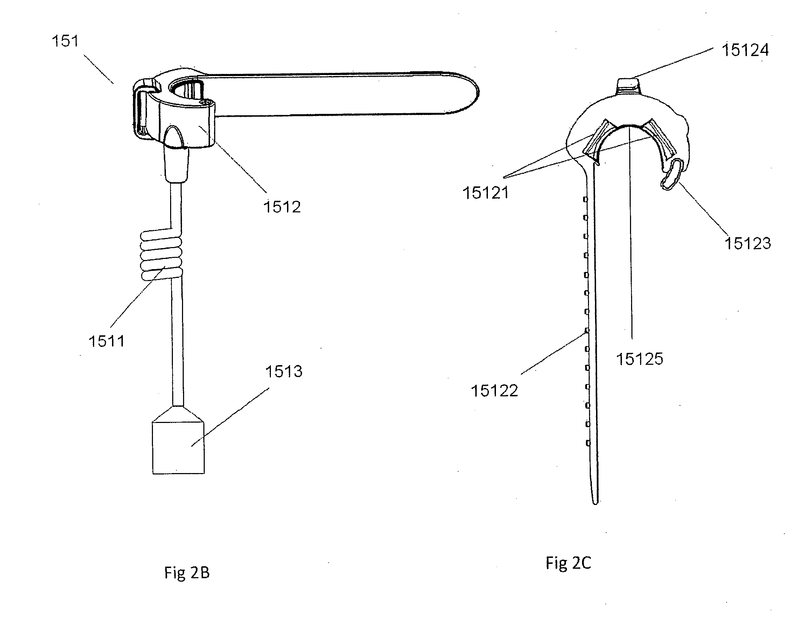

Fig. 1 shows a top view of the pulse sensor of the present invention. An optical finger probe 151 used for measuring the pulse signals is connected to the pulse sensor 1. An ON/OFF switch 152 is used for turning on or off the power of pulse sensor 1. Two LEDs 153 can indicate the status of wireless transmission and other relevant information. An emergency button 154 can be pressed and activate the emergency call function by anyone who needs help or aid. A pair of ear-like slots 155 is used for fixing the pulse sensor on user's wrist with a wristband. Shown in Fig. 2A, an optical finger probe 151 is worn on a user's finger and connected to the pulse sensor 1. The optical finger probe 151 is designed for long-term wearing with several specialized features. Referring to Fig. 2B, the soft tip 1512 made in a ring-like configuration can be worn on finger phalanx. This feature reduces disturbances during monitoring in daily life and sleeping. A coiled cable 1511 between soft tip 1512 and adapter 1513 is to increase tidiness and flexibility. Fig. 2C shows the cross-section of the top view of soft tip 1512. The optical sensing apparatus within the optical finger probe 151 is a plethysmographic sensor consisting of light emitting and detecting elements. There is a well 15121 in the inner side of soft tip 1512 for embedding the optical sensing apparatus consisting of photodiode 112 and LED 111. The curved structure 15125 made for fitting finger shape can enhance the contact between finger and optical sensing apparatus. The strap 15122 with bulged stripe is used for adjusting the size of the soft tip 1512. By inserting the strap 15122 into the plastic buckle 15123, soft tip becomes a closed configuration, like a ring structure. After wearing it on, adjusting the inner ring size can be easily done by stretching and loosing the strap 15122. The overhung strap 15122 can be tied with the latch 15124 if it is too long.

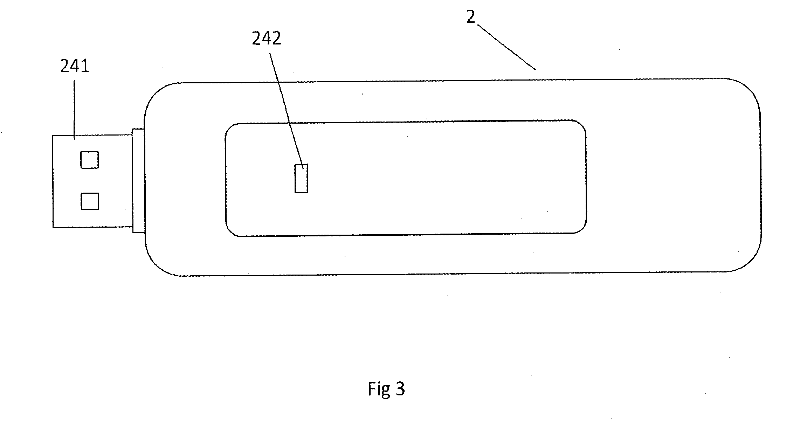

Please refer to Fig. 3, which is a top view of the USB receiver of the present invention. A LED 242 is used to indicate a wireless transmission status and other relevant information. A USB socket 241 is used for connecting a USB receiver 2 and a monitoring computer 3. A monitoring computer 3 runs the software to display the relevant physiological data.

Please refer to Fig. 4, which is a block diagram of the wireless optical pulsimetry system for a healthcare environment of the present invention. The physiological signals from a pulse sensor 1 and a multi vital signs recorder 4 are transmitted to a USB receiver 2 by wireless communication. The USB receiver 2 can receive the pulse rate from the pulse sensor 1 and use embedded wireless signal strength indicator to estimate the distance between the pulse sensor 1 and the USB receiver 2. The physiological signals and location information are transmitted from a USB receiver 2 to a monitoring computer 3. A USB extender or wireless repeater can be used to extend the transmission distance from USB receiver 2 to monitoring computer 3.

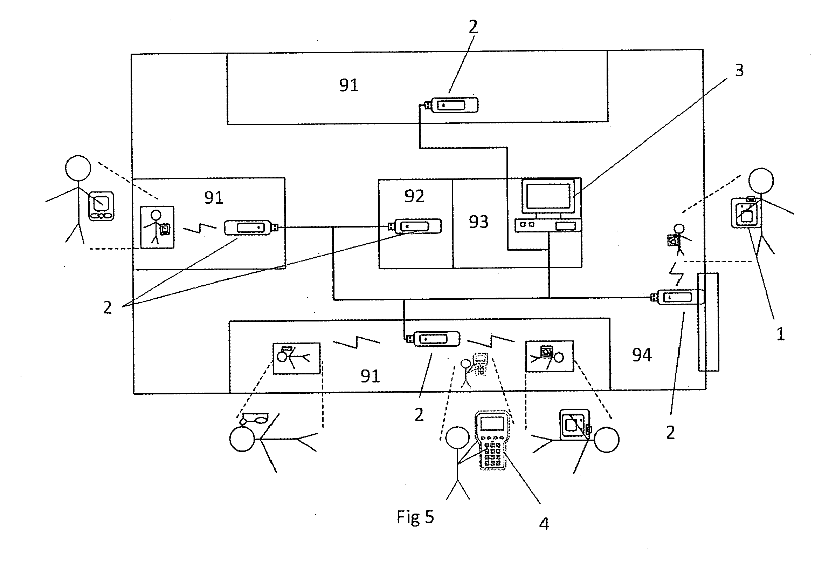

Please refer to Fig. 5 which is a schematic view of the wireless optical pulsimetry system used in a healthcare environment of the present invention. A monitoring computer 3 is disposed within a base station 93. USB receivers 2 can be disposed at a ward 91, a toilet 92, an exit 94 and a basement respectively. When a person wearing the pulse sensor 1 approaches the exit 94, a USB receiver 2 disposed at the exit 94 will receive a pulse signal from the user and then transmit the location, pulse rate information of the user to a monitoring computer 3. Caretakers in the ward 91 can use a multi vital signs recorder 4 to transmit vital signs to a monitoring computer 3 through a USB receiver 2.

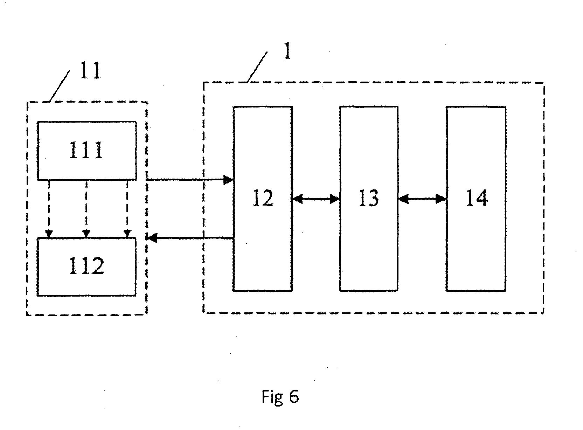

Please refer to Fig. 6, which is a block diagram of the pulse sensor of the present invention. In the pulse sensor 1, a LED 111 and a photodiode 112 of the physiological signal module 11 are used to obtain the pulsatile signal by means of sensing infrared absorption changes due to vascular volume change in the finger. The sensed pulsatile signal is filtered and amplified by a 4-stage pulsatile signal amplifier circuit 12. The amplified pulsatile signal is calculated by an algorithm through a microprocessor controlling module 13 to get the pulse rate and other relevant information. The pulse rate and other relevant information received from the microprocessor controlling module 13 are transmitted to a USB receiver 2 through a wireless transceiver module 14 based on Zigbee technology.

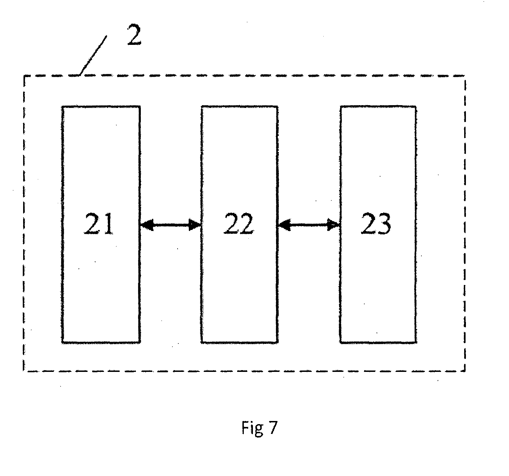

Please refer to Fig. 7, which is a block diagram of the USB receiver of the present invention. The pulse rate and other relevant information received by wireless transmission module 23 are processed and controlled by a microprocessor controlling module 22 and then transmitted to monitoring computer 3 via a USB interface module 21. The pulse rate and other relevant information are displayed on the screen of the monitoring computer 3.

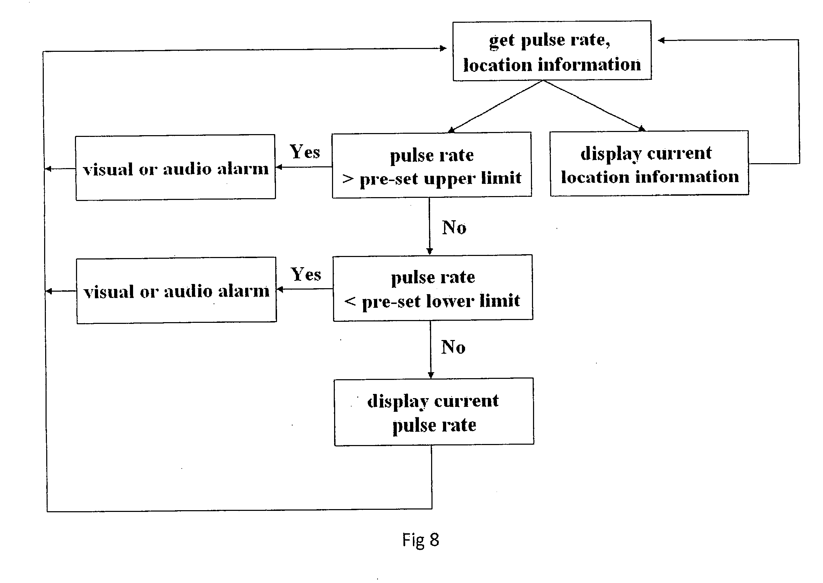

Please refer to Fig. 8, which is a flowchart of physiological signal processing software of the present invention wherein the physiological signal is a pulse rate. The software can display the pulse rate data, location information, movement of the pulse sensor, status of a wireless signal, and profile of the users more than 100 patients. Firstly, the pulse rate and location information are gotten. Then, the software will display the current location of a user and check if the pulse rate is above the upper limit or below the lower limit. If pulse rate exceeds the preset limit, the software can use an adjustable audible and visual alarm to raise the user's attention.

The advantages and non-obviousness of the present invention are as follows:
1. The monitoring of pulse rate of the users or patients is in real-time manner. For every 3 seconds, a pulse rate of each patient is displayed on the monitoring computer continuously.
2. The wireless optical pulsimetry system of the present invention can save energy of the secondary battery and can work and last for at least 5 days because it uses Zigbee technology.
3. The frame data of the Zigbee technology includes a sensor number of the USB receiver and the pulse rate data of the patients only.
4. The location information of each of the patients can be known by the monitoring computer.
5. The wireless optical pulsimetry system of the present invention can save labours.

Finding of location of a user is by sensing a highest strength of one of the receivers. For example, receiver (a) has a strength of 2.1, receiver (b) has a strength of 3.7 and receiver (c) has a strength of 0.5 for user RR. From the strength data of the user RR, we know that user RR is located near receiver (b), which shows a highest strength of 3.7.

## Claims

1. A wireless optical pulsimetry system for a healthcare environment comprising:
a pulse sensor for measuring a pulse rate of a user to transmit a pulse signal;
a USB receiver for receiving the pulse signal wirelessly from one or more pulse sensors and transmitting localization information of a user who wears the pulse sensor to a monitoring computer by transmitting means; and
a monitoring software running on the monitoring computer for displaying the pulse rate and the localization information of a user by display means.

2. The wireless optical pulsimetry system as claimed in claim 1, wherein the pulse sensor comprises:
an optical finger probe is used for collecting a pulsatile signal of the user;
a 4-stage pulsatile signal amplifier circuit for amplifying the pulsatile signal obtained from the optical finger probe;
a microprocessor for processing the pulsatile signal, calculating a pulse rate from the pulsatile signal, and transmitting the pulse rate; and
a wireless transceiver module for receiving the pulse rate from the microprocessor and for wireless transmitting the pulse rate to the USB receiver.

3. The wireless optical pulsimetry system as claim in claim 2, wherein the optical finger probe comprises:
a coiled cable is retractable and flexible for fitting different hand sizes of the user;
a soft tip worn on a finger of the user as a ring for measuring the pulsatile signal of the user; and
a adaptor for fixing the coiled cable on the pulse sensor.

4. The wireless optical pulsimetry system as claim in claim 3, wherein the soft tip comprises:
an optical sensing apparatus functioning as a plethysmographic sensor consisting of light emitting and detecting elements;
a well providing a space for the optical sensing apparatus embedded;
a strap with bulged stripe for adjusting the ring size of the soft tip;
a plastic buckle for fixing the strap;
a latch for tying up the strap overhung; and
a curved structure for fitting a shape of the finger to enhance the contact between the optical sensing apparatus and the finger.

5. The wireless optical pulsimetry system as claim in claim 4, wherein the optical sensing apparatus provides real-time pulsatile signal to the 4-stage pulsatile signal amplifier circuit by means of sensing infrared absorption changes due to vascular volume change in the finger.

6. The wireless optical pulsimetry system as claimed in claim 1, wherein collecting of pulse rate wirelessly from one or more pulse sensors is based on Zigbee technology.

7. The wireless optical pulsimetry system as claimed in claim 6, wherein frame data of the Zigbee technology includes a sensor number of the pulse sensor and the pulse rate data.

8. The wireless optical pulsimetry system as claimed in claim 1, wherein the transmitting means comprises:
a pulse sensor for sending the pulse rate to any USB receiver in its wireless coverage;
a USB receiver for receiving the pulse rate from the pulse sensor and using an embedded wireless signal strength indicator to estimate the distance between the pulse sensor and the USB receiver; and
a USB extender or wireless repeater for transmitting the information from the USB receiver to the monitoring computer.

9. The wireless optical pulsimetry system as claimed in claim 1, wherein the display means comprise:
a display software for displaying the pulse rate data, location information, movement of the pulse sensor, status of a wireless signal, and profile of the user;
an adjustable audible and visual alarm for raising the user attention when the pulse rate data exceeds a pre-set limit or ; and
a naming function device for setting the localization information of the USB receiver.

10. The wireless optical pulsimetry system as claimed in claim 1, wherein the healthcare environment includes a hospital, nursing home, home, or other healthcare facilities.

11. The wireless optical pulsimetry system as claimed in claim 1, further comprises a first-line screening function used in healthcare environments for finding a user with abnormal pulse rate data that might be caused by complex physiological abnormality.

12. The wireless optical pulsimetry system as claimed in claim 1, further comprises a multi vital signs recorder for collecting and transmitting multiple vital signs to the monitoring computer.

13. The wireless optical pulsimetry system as claimed in claim 1, wherein the wireless optical pulsimetry system is a real-time wireless optical pulsimetry system for monitoring the user in a real-time manner.
